# EUROPEAN PATENT SPECIFICATION

(11) **EP 2 743 263 B1**
(45) Date of publication and mention of the grant of the patent: **03.01.2018**
(21) Application number: 13193907.6
(22) Date of filing: 21.11.2013
(51) Int. Cl.: C07D 279/28

(54) **AN IMPROVED PROCESS FOR THE PREPARATION OF LEVOMEPROMAZINE MALEATE**
VERBESSERTES VERFAHREN ZUR HERSTELLUNG VON HERSTELLUNGSPROZESS FÜR LEVOMEPROMAZIN-MALEAT
PROCÉDÉ AMÉLIORÉ POUR LA PRÉPARATION DE LÉVOMÉPROMAZINE MALÉATE

(30) Priority: 29.11.2012 IN 3390MU2012
(43) Date of publication of application: 18.06.2014
(73) Proprietor: ZCL Chemicals Ltd., 400 059 Mumbia, Maharashtra (IN)
(72) Inventor: AGARWAL, Nand Lal, 393002 Ankleshwar, Bharuch District, Gujarat (IN); MISTRI, Pranav Popatlal, 393002 Ankleshwar, Bharuch District, Gujarat (IN); PATEL, Nitin Maganbhai, 393002 Ankleshwar, Bharuch District, Gujarat (IN)
(74) Representative: Gislon, Gabriele

(56) References cited:
- US-A- 2 837 518
- US-A- 4 798 895
- DATABASE CAPLUS [Online] CHEMICAL ABSTRACTS SERVICE, COLUMBUS, OHIO, US; PATAKI, SANDOR ET AL: "Resolution of 2-methoxy-10-(2-methyl-3- dimethylaminopropyl)phenothiazine", XP002723181, retrieved from STN Database accession no. 1970:132762 & HU 157 158 19 3 June 1958 (1958-06-03)
- DATABASE CAPLUS [Online] CHEMICAL ABSTRACTS SERVICE, COLUMBUS, OHIO, US; WROTEK, JERZY ET AL: "(+)- and (-)-2-Methoxy-10-(3-dimethylamino-2- methylpropyl)phenothiazines possibly as acid maleates", XP002723182, retrieved from STN Database accession no. 1974:3541 & PL 66 636 19 (INSTYTUT FARMACEUTYCZNY) 31 July 2013 (2013-07-31)
- DATABASE CAPLUS [Online] CHEMICAL ABSTRACTS SERVICE, COLUMBUS, OHIO, US; JAKFALVI, ELEMER ET AL: "(-)-10-(3-Dimethylamino-2-methylpropyl)-2 -methoxyphenothiazine maleate", XP002723183, retrieved from STN Database accession no. 1985:203977 & HU 32 813 A2 (EGYT GYOGYSZERVEGYESZETI GYAR, HUNG.) 28 September 1984 (1984-09-28)
- DATABASE CAPLUS [Online] CHEMICAL ABSTRACTS SERVICE, COLUMBUS, OHIO, US; TOKAR, GEZA ET AL: "Resolution of 3-methoxy-10-(2-methyl-3- dimethylaminopropyl)phenothiazine", XP002723184, retrieved from STN Database accession no. 1969:87834 & HU 155 314 19 (EGYESULT GYOGYSZER ES TAPSZERGYAR) 22 November 1968 (1968-11-22)
- DATABASE CAPLUS [Online] CHEMICAL ABSTRACTS SERVICE, COLUMBUS, OHIO, US; FOGASSY, ELEMER: "Optically active 3-methoxy-10-(2-methyl-3- dimethylaminopropyl)phenothiazine", XP002723185, retrieved from STN Database accession no. 1965:472057 & HU 152 208 19 (EGYESULT GYOGYSZER ES TAPSZERGYAR) 23 November 1964 (1964-11-23)
- DATABASE CAPLUS [Online] CHEMICAL ABSTRACTS SERVICE, COLUMBUS, OHIO, US; DOROGHAZI, MRS. PAL ET AL: "Phenothiazines", XP002723186, retrieved from STN Database accession no. 1975:410105 & HU 9 067 19 (E. GY. T. GYOGYSZERVEGYESZETI GYAR) 28 November 1974 (1974-11-28)
- DATABASE CAPLUS [Online] CHEMICAL ABSTRACTS SERVICE, COLUMBUS, OHIO, US; KAZANKOV, M. V. ET AL: "Alkylation method for preparing 10-(3-dimethylamino-2-methylpropyl)-2-meth oxyphenothiazine from 2-methoxyphenothiazine and 1-chloro-3-dimethylamino-2-methylpropane using crown ether catalysts", XP002723187, retrieved from STN Database accession no. 2004:670752 & RU 2 233 274 C1 (FGUP "GOS. NAUCHNYI TSENTR "NAUCHNO-ISSLEDOVATEL'SKII INSTITUT ORGANIC) 27 July 2004 (2004-07-27)

## Description

### FIELD OF THE INVENTION

The present invention relates to an improved and industrially applicable process for the preparation of levomepromazine maleate of formula I,

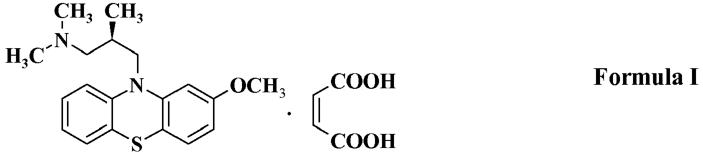

### BACKGROUND OF THE INVENTION

Levomepromazine or (-)-10-(3-dimethylamino-2-methylpropyl) -2-methoxyphenothiazine is a therapeutic agent which is especially useful as a tranquillizer, antiemetic, neuroleptic and analgesic.

Levomepromazine is used for the treatment of psychosis, particular those of schizophrenia, and manic phases of bipolar disorder. Levomepromazine is also used at lower doses for the treatment of nausea and insomnia. In Europe it is approved for the treatment of chronic idiopathic pain. Levomepromazine is increasingly being prescribed and valued worldwide in Palliative Care Medicine. In Palliative Care Medicine Levomepromazine is valued for its multimodal action. It is now being used widely in palliative care to treat intractable nausea or vomiting, and for severe delirium/ agitation in the last days of life. Palliative Care Physicians will commonly prescribe it via subcut syringe drivers in combination with more potent opiate analgesics such as hydromorphone. It is also used orally in this regard.

In Europe it has been marketed for decades as Neurocil and Nozinan (manufactured by Sanofi-Aventis). Nozinan is also available in Canada. Nozinan is available in round tablets of 5, 25 or 100 mg. (reference from Wikipedia, http://www.rxlist.cn, http://www.palliativecareguidelines.scot.nhs.uk/documents/Levomepromazine.pdf) Levomepromazine and compounds were first disclosed in US patent 2,837,518. According to said patent, levomepromazine prepared by the condensation of 2-methoxyphenothiazine and 3-dimethylamino-2-methylpropyl chloride in xylene in the presence of sodamide at quite higher temperature at about 130°C for long time about 20hours which is further proceeded for the acid-base treatment. In that process, the above obtained reaction mixture is cooled and treated with water & methanesulphonic acid to form the salt and the aqueous layer is washed with ether. The layer is treated with aqueous sodium hydroxide and then extracted with ether followed by high vacuum distillation to get racemic base of (±)-10-(3-dimethylamino-2-methylpropyl)-2-methoxyphenothiazine. Further the obtained base dissolved in acetone and ethereal hydrogen chloride was added to obtain hydrochloride salt of (±)-10-(3-dimethylamino-2-methylpropyl)-2-methoxyphenothiazine. The obtained compound is treated with d-tartaric acid in isopropanol. The product is filtered off to get (+)-10-(3-dimethylamino-2-methylpropyl) -2-methoxyphenothiazine d-tartrate. The mother liquor obtained after the filtration, treated with alcoholic potash followed by seeding with (-)-10-(3-dimethylamino-2-methylpropyl)-2-methoxyphenothiazine to get the precipitation of (-)-10-(3-dimethylamino-2-methylpropyl)-2-methoxyphenothiazine base, which is then filtered and treated with water and ether followed by decantation and evaporation. Thereafter, (-)-10-(3-dimethylamino-2-methylpropyl)-2-methoxy- phenothiazine is treated with maleic acid in ethanol to get levomepromazine maleate. The above disclosed process is silent about HPLC purity of the product.

One of the major disadvantages of this process is use of sodamide which is very much hazardous as it may form explosive peroxides by reacting with air hence sodamide needs to be stored in a tightly closed container, under an atmosphere of nitrogen gas and it is very difficult to handle from the safety point of view at large scale. Secondly, sodamide is corrosive to the skin, eyes and mucous membranes. Thirdly, it is observed that during use of sodamide in the reaction, huge quantity of ammonia gas liberated which is not in the favor of environment (reference from material safety datasheet of sodamide). Furthermore, the disclosed process involves solvent extraction at various stages, salt formation and breaking, use of potash and use of ether is hazardous, tedious, and cumbersome at the plant level. Hence above disadvantages of the disclosed process make it unattractive.

Other reported processes to prepare levomepromazine by resolution of (±)-10-(3-dimethylamino-2-methylpropyl)-2-methoxyphenothiazine with various resolving agents, e.g. tartaric acid (Hungarian Patent HU 32,813, Chem. Abstr. 102 203977 w; Hungarian Patent HU 157,158, Chem. Abstr. 72 132762 w) or dibenzoyl-D-tartaric acid (Hungarian Patent HU 152,208, Chem. Abstr. 63 13279 b), or with (-)-O-diacetyltartaramic acid (Polish Patent POL 66,636, Chem. Abstr. 80 3541 q). However, these processes do not always lead to satisfactory yields as well as purity, or else they require the use of excess quantities of resolving agent and solvents which may sometimes be obtainable only with difficulty. The above cited patents are silent about the purity as well as chiral purity of the levomepromazine or its salts thereof. Hence these processes are not economical, environment friendly and industrially viable.

US patent 4,798,895 discloses the process for preparing levomepromazine hydrogen maleate prepared by resolving (±)-10-(3-dimethylamino-2-methylpropyl)-2-methoxyphenothiazine with (-)-di(p-toluoyl)-L-tartaric acid to produce the neutral salt of the undesired isomer and then adding maleic acid to the mother liquors to precipitate the levomepromazine hydrogen maleate. The process disclosed in the patent is tedious, lengthy and time consuming. Also the disclosed process is silent about the purity of the product.

Thus, present invention fulfills the need of the art and provides an improved and industrially applicable process for preparation of levomepromazine maleate, which provides levomepromazine and the intermediates useful for its synthesis in high overall yield and purity.

### OBJECTIVE OF THE INVENTION

The principal objective of the present invention is to provide an efficient and industrially advantageous process for preparation of levomepromazine maleate.

Another objective of the invention is to provide a process for preparation of levomepromazine maleate avoiding the use of sodamide. Described herein is an environment friendly process for the preparation of intermediates of levomepromazine maleate.

Another objective of the invention is to provide an efficient, improved and industrially advantageous process for preparation of levomepromazine maleate which is conveniently applicable to industrial scale and avoiding use of huge volumes of solvents and excess quantities of resolving agents.

Another objective of the present invention is to provide a process for isolation of solid levomepromazine maleate from reaction mass by filtration or centrifugation.

Yet another objective of the present invention is to provide levomepromazine maleate having high purity and yield.

### SUMMARY OF THE INVENTION

Accordingly, the present invention provides an improved process for the preparation of levomepromazine maleate of formula I, 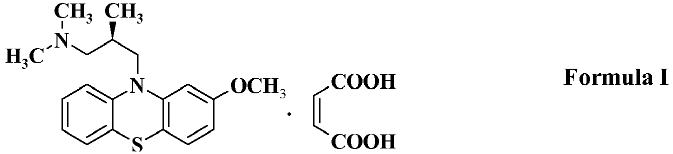 which proves to be efficient and industrially viable. The process comprises the steps of:
a). condensing the compound of formula II, 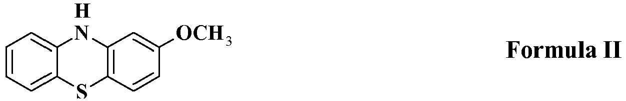 with compound of formula III, 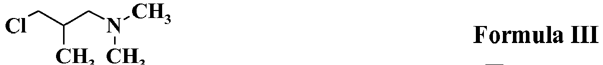 in the presence of an alkali hydroxides in mixture toluene and dimethylsulfoxide to form a compound of formula IV; 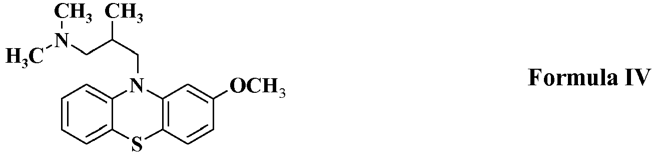
b). isolating the compound of formula IV by filtration;
c). distilling the compound of formula IV at high vacuum;
d). treating the obtained compound of formula IV from step c) with (-)-dibenzoyl-L-tartaric acid monohydrate as resolving agent in a solvent;
e). filtering the reaction mixture;
f). adding maleic acid in mother liquor;
g). precipitating levomepromazine maleate from the mother liquor; and
h). optionally purifying levomepromazine maleate of formula I, wherein the levomepromazine maleate obtained is having purity greater than 99.7%. Accordingly, the present invention provides an environment friendly process for the preparation of compound of formula IV, process comprises the step of: condensing compound of formula II, with compound of formula III, in the presence of alkali hydroxides in mixture of suitable solvent to form a compound of formula IV.

Accordingly, the present invention provides an improved process for the preparation of compound of formula I, process comprises the step of: the resolution of compound of formula IV by treating compound of formula IV with resolving agent and converting to the compound of formula I.

Accordingly, the present invention provides an improved process for the preparation of pharmacopoeial grade levomepromazine maleate.

### DETAILED DESCRIPTION OF THE INVENTION

As used herein, the term "ambient temperature" describing common indoor temperatures usually falls in the range of 25 to 35°C.

As used herein, the term "reflux temperature" describing the boiling temperature of solvents.

As used herein, the term "Undesired isomer" is free base of (+)-10-(3-dimethylamino-2-methylpropyl)-2-methoxyphenothiazine.

All ranges recited herein include the endpoints, including those that recite a range "between" two values. Terms such as "about", "generally" and the like are to be construed as modifying a term or value such that it is not an absolute. Such terms will be defined by the circumstances and the terms that they modify as those terms are understood by those skill in the art. This includes, at very least, the degree of expected experimental error, technique error and instrument error for a given technique used to measure a value.

The present invention provides an improved and efficient process for the preparation of levomepromazine maleate of formula I.

According to the embodiment of the invention provides an industrially advantageous process for preparation of levomepromazine maleate starting from compound of formula II.

### Stage 1:

The compound of formula II can be reacted with compound of formula III in the presence of alkali hydroxides in mixture of toluene and dimethylsulfoxide to form a compound of formula IV. Generally, the reaction involves the condensation of compound of formula II with compound of formula III in the presence of alkali hydroxides. Suitable alkali hydroxides include sodium hydroxide, potassium hydroxide, lithium hydroxide and the like. Preferably, potassium hydroxide. The reaction mixture is refluxed for 1 to 12 hours, preferably for 2-3 hours, more preferably till the completion of the reaction. The reaction completion is monitored by suitable chromatographic techniques such as high pressure liquid chromatography (HPLC) or thin layer chromatography (TLC). The compound of formula IV can be isolated from the reaction mixture by suitable techniques such as filtration or centrifugation and the like. The process resulted in desired compound in high purity and yield.

Particularly, compound of formula II is reacted with potassium hydroxide in the mixture of toluene and dimethyl sulphoxide at reflux temperature for 2 hours to form the potassium salt of compound of formula II. Further, it is reacted with compound of formula III which is in toluene, at reflux temperature till the completion of the reaction, confirmed by thin layer chromatography. During the reaction water is removed continuously. The reaction mixture is cooled to ambient temperature and treated with water. Thus obtained two layers are separated. The organic layer is washed with water and treated with 20% sodium chloride solution. Then after the organic layer is extracted with 1N hydrochloric acid solution and basified with 20% sodium hydroxide till the pH 8.0 to 9.5 is obtained. The reaction mixture is stirred for about 1 hour. The solid obtained is filtered and dried. Further washed with water and dried to get the compound of formula IV.

The main advantage of the present invention is to develop environment friendly process for the preparation of the compound of formula IV. In another way the present invention is advantageous as it circumvents the need of sodamide during the preparation of compound of formula IV. In addition to this, the prior art process involves longer and tedious removal of sodamide from the reaction mixture as well as huge quantity of ammonia liberates during the reaction which can cause eye irritation, suffocation like side effects. The prior art process for the preparation of compound of formula IV is time consuming, tedious and harmful to the environment. It is known to scientist's community that use of sodamide is difficult to handle at the large scale, explosion may occur in the plant and it may be the life threatening. So, the present invention opens the way to prepare levomepromazine maleate without using sodamide at the intermediate stage. Also the present invention resulted in high purity and yield of compound of formula IV; hence the present invention is environment friendly and industrially viable.

### Stage 2:

The compound of formula IV can be purified by high vacuum distillation. The process can be performed at high vacuum less than 266.644 Pa (2.0mmHg), preferably 133.322 Pa (1.0mmHg) and temperature range at about 90 to 240°C.

The main advantage of high vacuum distillation affords the very good quality. It avoids use of large volume of solvents for the purification. Hence, the process is environment friendly as well as economically cost effective. The present invention avoids the acid-base treatment which differs from the prior art processes and leads to the ease of the process. The compound of formula IV obtained by the present invention have purity more than 95%, preferably more than 97%, more preferably greater than 99%.

### Stage 3:

Generally, the reaction involves compound of formula IV can be treated with (-)-dibenzoyl-L-tartaric acid monohydrate as resolving agent at ambient temperature in suitable solvent. Suitable solvent that include alcohols such as methanol, ethanol; aliphatic ketones such as acetone, diethyl ketone, methyl isobutyl ketone; ethers such as tetrahydrofuran, 1,2-dimethoxy ethane, 1,2-diethoxy ethane, 1,4-dioxane; alkyl nitrile such as acetonitrile, propionitrile and the like or mixture thereof. The reaction mixture is stirred for 20 minutes to 8 hours, preferably till the undesired isomer salt is precipitated.

The mother liquor obtained by the filtration can be treated with the maleic acid in suitable solvent that include alcohols such as methanol, ethanol; aliphatic ketones such as acetone, diethyl ketone, methyl isobutyl ketone; ethers such as tetrahydrofuran, 1,2-dimethoxy ethane, 1,2-diethoxy ethane, 1,4-dioxane; alkyl nitrile such as acetonitrile, propionitrile and the like or mixture thereof. The reaction mixture is stirred or maintained for few minutes to few hours. Compound of formula I can be isolated from the reaction mixture by suitable techniques such as filtration, centrifugation, crystallization and the like.

Particularly, compound of formula IV is reacted with the (-)-dibenzoyl-L-tartaric acid monohydrate in a solvent preferably acetone at the ambient temperature. The reaction mixture is stirred for 20-30 minutes until the solid precipitation of (-)-dibenzoyl-L-tartaric acid salt of undesired isomer is obtained and removed by filtration.

The obtained mother liquor is made five volumes exactly by adding excess acetone to the filtrate. The solution of maleic acid in one volume acetone is added to the mother liquor. The solid material is precipitated out spontaneously. The reaction mass is maintained for 1 hour at ambient temperature then cooled to 10°C and maintained for 1 hour. Further the reaction mass is maintained for 1 hour at -10°C. After completion of the 3-4 hours maintenance, the reaction mass is filtered at about -10°C temperature to get the levomepromazine maleate of formula I. Levomepromazine maleate obtained from the present invention is highly pure and free from undesired impurities. The compound of formula I obtained by the present invention has purity greater than 99.7%.

The main advantage and inventiveness of this reaction is to get the purer product from the mother liquor solution. The scientist of this invention observed that they are getting the product in high yield and purity than the reported in the prior art. The process reported in Hungarian Patent HU 152,208, Chem. Abstr. 63 13279 b used dibenzoyl-D-tartaric acid for the resolution of the racemic compound and converted to maleate salt. Patent involves five steps preparation include chiral acid salt formation followed by breaking the salt by adding ammonium hydroxide. Further the mother liquor obtained by the filtration is distilled off to get residual oil which is then treated with water and ammonium hydroxide to obtain the crude base of desired isomer. The crude base is recrystallized in five volume ethanol. The pure free base converted to acetate salt and exchanged with maleic acid to get levomepromazine maleate. The process disclosed in HU'208 is very much tedious, time consuming, uneconomic and industrially not viable. Hence using (-)-dibenzoyl-L-tartaric acid monohydrate for the resolution is more advantageous over the prior art reported.

Another advantage of the present invention is to circumvent the use of solvents for the extraction and purification; it makes the process cost effective on a commercial scale. Still another advantage but not the last involves easy isolation of compound of formula I from the reaction mixture by employing filtration or centrifugation. The process avoids the need of extraction with large volume of solvent or liquid-liquid extraction or column chromatography, therefore it makes the process cost effective and industrially viable.

In another alternate way, present invention provides a process for recovery of levomepromazine from the above undesired isomer salt. Thus, the (-)-dibenzoyl-L-tartaric acid salt of undesired isomer can be further racemize by the known techniques reported in the art for the different compounds and further proceed for the resolution as describe above. This leads to increase the total yield of the final product. It is industrially advantageous to recover the product.

The invention is further defined by reference to the following examples describing in detail by the preparation of the compounds of the invention. It will be apparent to those skilled in the art that many modifications, both to materials and methods, may be practiced without departing from the scope of the invention.

### EXAMPLES:

### Stage-1

### Preparation of formula (IV):

To a suspension of 2-methoxyphenothiazine compound of formula II (100gm, 0.4366mole) in toluene (300ml), dimethyl sulphoxide (DMSO) (60ml) and potassium hydroxide (48.89gm, 0.8732mole) were added. The reaction mixture was maintained for 2 hours at reflux temperature to form potassium salt of 2-methoxyphenothiazine. 3-dimethylamino-2-methylpropyl chloride (66.9gm, 0.4937mole) in solution of toluene (150ml) was added into the reaction mixture at the reflux temperature. The reaction mixture was maintained till the absence of starting material. Then, the reaction mixture was cooled to ambient temperature and water (200 ml) was charged. The organic layer was separated, washed with water (200mlx2) and 20% NaCl (200 ml) solution. The product was extracted with 1N HCl (300mlx2) and basified by using 20% sodium hydroxide (120 ml) till pH 8.0-9.5 was obtained. The reaction mass was stirred for 1 hour, filtered and dried. The obtained solid was washed with water (200mlx3) and suck dried. The wet cake was dried at temperature 50 to 60°C to get 125 to 135gm of the title compound. HPLC purity 90-94%

### Stage-2

### Purification of formula (IV) from stage 1:

In a high vacuum assembly compound of formula (IV) (120 gm) was charged and material was heated to temperature 90 to 100°C. Then distillation was performed at 200 to 240°C temperature under vacuum at 133.322 Pa (1.0mmHg). Distilled base was cooled to ambient temperature. The solid material was obtained 96 to 108gm. HPLC purity 99.0%

### Stage-3

### Preparation of Levomepromazine maleate of formula (I)

(±)-10-(3-dimethylamino -2-methylpropyl)-2-methoxy phenothiazine of formula IV of stage 2 (100 gm, 0.3030 mole), (-)-dibenzoyl-L-tartaric acid monohydrate (114.3 gm, 0.3030 mole) were added in acetone (400 ml). The reaction mixture was stirred for 20 minutes until the solid precipitation of undesired isomer salt. The precipitated material was separated by filtration and washed with acetone (100 ml). The above obtained mother liquor was made five volume by acetone followed by addition of the solution of maleic acid (21.0 gm, 0.1818 mole) in acetone (100 ml). The solid material was fall out spontaneously. The reaction mass was maintained for 1 hour at ambient temperature, 1 hour at 10°C and 1 hour at -10°C. The reaction mass was filtered at temperature of - 10°C followed by washing with acetone (100mlx3) to obtain levomepromazine maleate (50 gm) about 74% yield. HPLC purity 99.75%, Specific optical rotation [α]_{d}²⁰ = -7.7°C (c=5, dimethylformamide)

## Claims

1. An improved process for the preparation of levomepromazine maleate compound of formula I 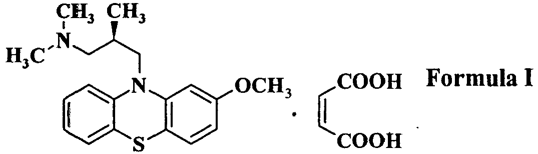 comprising the steps of:
a). condensing the compound of formula II, 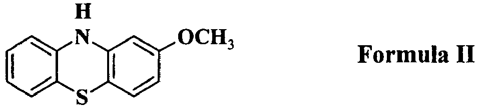 with the compound of formula III, 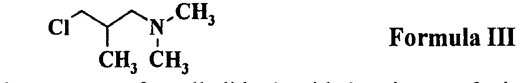 in the presence of an alkali hydroxide in mixture of toluene and dimethylsulfoxide to form a compound of formula IV; 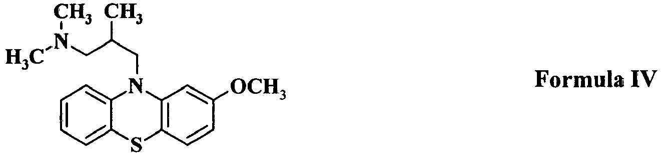
b). isolating the compound of formula IV;
c). distilling the compound of formula IV at high vacuum;
d). treating the compound of formula IV obtained from step c) with (-)-dibenzoyl-L-tartaric acid monohydrate as resolving agent in a solvent;
e). filtering off the reaction mixture;
f). adding maleic acid in the mother liquor of step e);
g). isolating levomepromazine maleate which precipitates from the mother liquor; and
h). optionally purifying levomepromazine maleate of formula I,
wherein the levomepromazine maleate obtained is having purity greater than 99.7%.

2. The process according to claim 1, wherein
in step a), said alkali hydroxide is selected from the group consisting of sodium hydroxide, potassium hydroxide and lithium hydroxide; and /or
in step c), said high vacuum is less than 266.644 Pa (2.0mmHg), preferably at 133.322 Pa (1.0mmHg) at the temperature range of 90-240°C.

3. The process according to claim 1, wherein said alkali hydroxide is potassium hydroxide.

## Patentansprüche

1. Ein verbessertes Verfahren zur Herstellung von Levomepromazinmaleat-Verbindung der Formel I 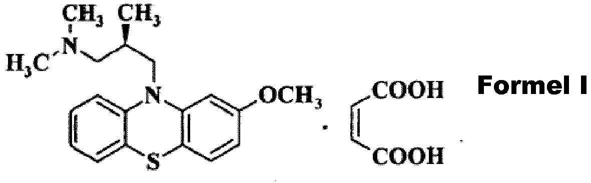 umfassend die Schritte:
a) Kondensation der Verbindung der Formel II, 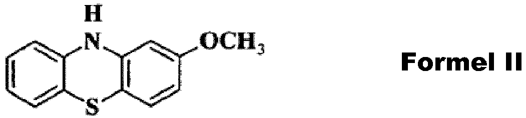 mit der Verbindung der Formel III, 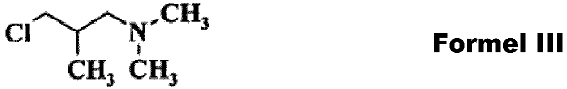 in Gegenwart von einem Alkalihydroxid in Mischung mit Toluol und Dimethylsulfoxid, um eine Verbindung der Formel IV zu bilden; 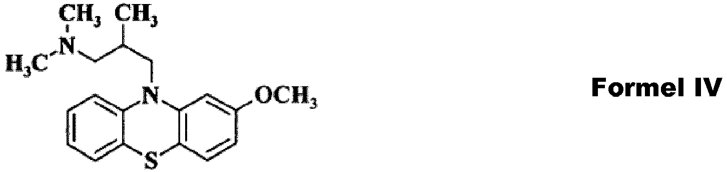
b) Isolierung der Verbindung der Formel IV;
c) Destillieren der Verbindung der Formel IV im Hochvakuum;
d) Behandeln der aus Schritt c) erhaltenen Verbindung der Formel IV mit (-)-Dibenzoyl-L-weinsäure-Monohydrat in einem Lösungsmittel;
e) Abfiltrieren der Reaktionsmischung;
f) Hinzufügen von Maleinsäure in der Mutterlauge von Schritt e);
g) Isolierung von Levomepromazinmaleat, das aus der Mutterlauge präzipitiert; und
h) gegebenenfalls Reinigen von Levomepromazinmaleat der Formel I,
wobei das Levomepromazinmaleat der Formel I mit einer Reinheit von mehr als 99,7 % erhalten wird.

2. Verfahren nach Anspruch 1, wobei in Schritt a) das Alkalihydroxid ausgewählt ist aus der Gruppe, bestehend aus Natriumhydroxid, Kaliumhydroxid und Lithiumhydroxid; und / oder in Schritt c) das Hochvakuum weniger als 266,644 Pa (2,0 mmHg), vorzugsweise bei 133,322 Pa (1,0 mmHg) im Temperaturbereich von 90-240 °C beträgt.

3. Verfahren nach Anspruch 1, wobei das Alkalihydroxid Kaliumhydroxid ist.

## Revendications

1. Procédé amélioré de préparation d'un composé maléate de lévomépromazine de formule I 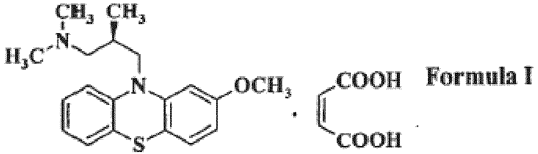 comprenant les étapes de :
a). condensation du composé de formule II, 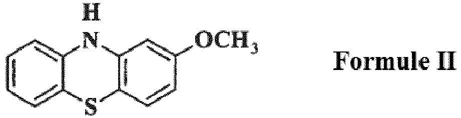 avec le composé de formule III, 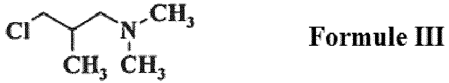 en présence d'un hydroxyde alcalin dans un mélange de toluène et de diméthylsulfoxyde pour former un composé de formule IV : 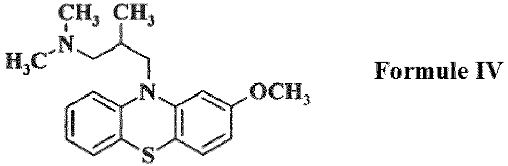
b). isolement du composé de formule IV ;
c). distillation du composé de formule IV sous vide poussé ;
d). traitement du composé de formule IV obtenu à l'étape c) avec de l'acide (-)-dibenzoyl-L-tartrique monohydrate en tant qu'agent de résolution dans un solvant ;
e). élimination du mélange de réaction par filtration ;
f). addition d'acide maléique à la liqueur mère de l'étape e) ;
g). isolement du maléate de lévomépromazine qui précipite de la liqueur mère ; et
h). facultativement purification du maléate de lévomépromazine de formule I,
le maléate de lévomépromazine obtenu ayant une pureté plus grande que 99,7 %.

2. Procédé selon la revendication 1, dans lequel
à l'étape (a), ledit hydroxyde alcalin est choisi dans le groupe consistant en l'hydroxyde de sodium, l'hydroxyde de potassium et l'hydroxyde de lithium ; et/ou
à l'étape c), ledit vide poussé est de moins de 266,644 Pa (2,0 mmHg), de préférence à 133,322 Pa (1,0 mmHg) dans la plage de températures de 90 à 240 °C.

3. Procédé selon la revendication 1, dans lequel ledit hydroxyde alcalin est de l'hydroxyde de potassium.
